Europäisches Patentamt

**European Patent Office**

**Office européen des brevets**

(19)

(11) Veröffentlichungsnummer: **0 240 840**

**A2**

(12) **EUROPÄISCHE PATENTANMELDUNG**

(21) Anmeldenummer: **87104383.2**

(22) Anmeldetag: **25.03.87**

(51) Int. Cl.⁴: **A61N 1/32**

(30) Priorität: **05.04.86 DE 3611537**

(43) Veröffentlichungstag der Anmeldung:
**14.10.87 Patentblatt 87/42**

(84) Benannte Vertragsstaaten:
**DE FR GB IT**

(71) Anmelder: **STS Elektronik**
**Buchenweg 44**
**D-2056 Glinde(DE)**

(72) Erfinder: **Steffen, Hartwig**
**Schlüskamp 14**
**D-2357 Bad Bramstedt(DE)**

(74) Vertreter: **Weisse, Jürgen, Dipl.-Phys. et al**
**Patentanwälte Dipl.-Phys. Jürgen Weisse**
**Dipl.-Chem. Dr. Rudolf Wolgast Bökenbusch**
**41 Postfach 11 03 86**
**D-5620 Velbert 11 Langenberg(DE)**

(54) **Vorrichtung zur Erzeugung von Strömen für die elektrotherapeutische Behandlung.**

㊗ Zur Erzeugung von Strömen für die elektrotherapeutische Behandlung werden die verstärkten Ausgangsströme zweier gegeneinander verstimmter Oszillatoren (10,12) auf Strompfaden (26,34), die einander im Behandlungsgebiet (36) kreuzen, in ein Gewebe eingeleitet. In dem Behandlungsgebiet (36) entstehen Ströme mit einer Schwebungsfrequenz. Eine Frequenzauswerterschaltung (42) liefert die Differenzfrequenz. Diese wird in einem auf-und abwärtszählenden Zähler (46) mit einer Sollfrequenz verglichen. Der Zählerstand wird in eine Spannung umgesetzt, welche die Frequenz des einen Oszillators (12) steuert. Es wird weiter eine Schaltung zur automatischen Einstellung des Arbeitspunktes der Schwebungsfrequenzregelung beschrieben.

**EP 0 240 840 A2**

## Vorrichtung zur Erzeugung von Strömen für die elektrotherapeutische Behandlung

Die Erfindung betrifft eine Vorrichtung zur Erzeugung von Strömen für die elektrotherapeutische Behandlung, bei welcher die verstärkten Ausgangsströme zweier gegeneinander verstimmter Mittelfrequenzoszillatoren auf Strompfaden, die einander in einem Behandlungsgebiet kreuzen, in ein Gewebe eingeleitet werden, so daß in dem Behandlungsgebiet Ströme mit einer Schwebungsfrequenz entstehen, enthalten:

(a) einen festfrequenten Oszillator und

(b) einen spannungsgesteuert-frequenzveränderlichen Oszillator mit einem Steuereingang.

Solche Vorrichtungen bezwecken in einem bestimmten Behandlungsgebiet Ströme mit einer verhältnismäßig niedrigen, veränderbaren Frequenz zu erzeugen, wobei in anderen Gebieten solche Ströme nicht auftreten sollen, sondern lediglich Ströme höherer Frequenzen im Mittelfrequenzenbereich. Dies wird dadurch erreicht, daß über zwei Paare von Elektroden Ströme über Strompfade eingeleitet werden, die sich in einem Behandlungsbereich kreuzen. In diesem Bereich treten dann Interfrequenzen oder Schwebungen auf, welche die gewünschte Differenzfrequenz besitzen. Außerhalb des Behandlungsgebietes sind die Ströme für sich allein wegen der höheren Frequenzen unwirksam.

Es sind verschiedene derartige Geräte bekannt (DE-PS 1 109 280, AT-PS 203 147). Bei den bekannten Geräten treten infolge Alterung, Bauteiltoleranzen und Temperatureinflüssen Frequenzschwankungen auf. Die Geräte müssen daher laufend nachgeeicht werden, um mit genau definierten, therapeutisch wirksamen Frequenzen arbeiten zu können.

Der Erfindung liegt demgemäß die Aufgabe zugrunde, eine Vorrichtung der eingangs genannten Art selbsteichend auszubilden, so daß eine exakte Einstellung der in dem Behandlungsgebiet auftretenden Differenzfrequenz sowie eine Konstanz der Grundfrequenzen beider Oszillatoren gewährleistet ist.

Erfindungsgemäß wird diese Aufgabe dadurch gelöst:

(c) die Ausgangsfrequenzen des festfrequenten Oszillators und des spannungsgesteuert-frequenzveränderlichen Oszillators auf eine Frequenzauswerterschaltung aufgeschaltet sind, die eine Signalfolge mit der Differenzfrequenz liefert,

(d) ein auf-und abwärtszählender Zähler an einem Aufwärtseingang mit einer Sollfrequenz und an einem Abwärtseingang mit der Differenzfrequenz von der Frequenzauswerterschaltung beaufschlagt ist,

(e) der Zählerstand des Zählers auf einen Digital-Analog-Wandler aufgeschaltet ist und

(f) der spannungsgesteuert-frequenzveränderliche Oszillator durch das analoge Ausgangssignal des Digital-Analog-Wandlers gesteuert ist.

In dem auf-und abwärtszählenden Zähler wird die zwischen den beiden Oszillatoren auftretende Differenzfrequenz mit einer üblicherweise kontrolliert veränderbaren Sollfrequenz verglichen. Bei einer Abweichung zählt der Zähler aufwärts oder abwärts. Der Zählerstand wird durch den Digital-Analog-Wandler in eine Spannung umgesetzt. Mit dieser Spannung wird die Frequenz des spannungsgesteuert-frequenzveränderlichen Oszillators so lange verändert, bis die Differenzfrequenz gleich der Sollfrequenz ist. Wenn dieser Zustand erreicht ist, tritt zu jedem aufwärtszuzählenden Impuls ein abwärtszuzählender Impuls auf, so daß der Zählerstand und damit die Spannung am Digital-Analog-Wandler unveränderbar bleibt.

Ausgestaltungen der Erfindung sind Gegenstand der Unteransprüche.

Ein Ausführungsbeispiel der Erfindung ist nachstehend unter Bezugnahme auf die zugehörige Zeichnung näher erläutert, die ein Blockschaltbild einer Vorrichtung zur Erzeugung von Strömen für die elektrotherapeutische Behandlung zeigt.

Die Vorrichtung zur Erzeugung von Strömen für die elektrotherapeutische Behandlung enthält einen festfrequenten Oszillator 10 sowie einen spannungsgesteuert-frequenzveränderlichen Oszillator 12. Der Oszillator 12 hat einen Steuereingang 14. Die Oszillatoren 10 und 12 liefern Folgen von Rechteckimpulsen. Deshalb ist dem Oszillator 10 ein Tiefpaßfilter 16 nachgeschaltet. Dem Oszillator 12 ist ein Tiepaßfilter 18 nachgeschaltet. Die Tiefpaßfilter 16 und 18 erzeugen aus den Rechteckimpulsen im wesentlichen sinusförmige Wechselspannungen. Die Wechselspannung von dem Tiefpaßfilter 16 wird mittels eines Leistungsverstärkers 20 verstärkt. Die verstärkte Wechselspannung liegt an einem ersten Paar von Elektroden 22,24 an, die einen Strompfad 26 in einem zu behandelnden Gewebe definieren. In entsprechender Weise wird die Wechselspannung von dem Tießpaßfilter 18 mittels eines Leistungsverstärker 28 verstärkt. Die verstärkte Wechselspannung liegt an einem zweiten Paar von Elektroden 30,32 an , die einen Strompfad 34 definieren. Der Strompfad 34 kreuzt den Strompfad 26 in einem Bereich 36. Wenn die Frequenzen der beiden Oszillatoren 10 und 12 unterschiedlich sind, dann tritt in diesem Bereich 36 eine Schwebung auf: Es werden dort und nur dort Ströme mit der Differenzfrequenz erzeugt. Wenn beispielsweise die Frequenz des Oszillators 10 den Wert 4 kHz und die Frequenz des Oszillators 12 den Wert 4,1 kHz, dann tritt im Bereich 36 ein Wechselstrom mit einer Frequenz von 100 Hz auf. Diese relativ niedrigen Frequenzen haben eine therapeutische Wirkung, während die Ströme mit 4 kHz bzw. 4,1 kHz im wesentlichen wirkungslos durch das Gewebe hindurchgehen. Die therapeutisch wirksamen Ströme werden daher lokalisiert nur dort erzeugt, wo sie benötigt werden. Es hat sich gezeigt, daß aus therapeutischen Gründen die Schwebungsfrequenz, also die Differenz der Frequenzen der beiden Oszillatoren 10 und 12 veränderbar sein muß. Es gilt also die Frequenzen der Oszillatoren 10 und 12 oder eines dieser Oszillatoren so zu steuern, daß die Differenzfrequenz einer vorgegebenen Sollfrequenz entspricht.

Bei der hier dargestellten Anordnung ist ein Mikroprozessor 38 vorgesehen. Der Mikroprozessor 38 dient zur Steuerung des Ablaufs in noch zu beschreibender Weise und erzeugt an einem Ausgang 40 auch die Sollfrequenz. Die Ausgangsfrequenzen des festfrequenten Oszillators 10 und des spannungsgesteuert-frequenzveränderlichen Oszillators 12 auf eine Frequenzauswerterschaltung 42 aufgeschaltet. Die Frequenzauswerterschaltung 42 liefert an einem Ausgang 44 eine Signalfolge mit der Differenzfrequenz $\Delta f = f_2 - f_1$.

Ein auf-und abwärtszählender Zähler 46 ist an einem Aufwärtseingang 48 mit der Sollfrequenz vom Ausgang 40 des Mikroprozessors 38 beaufschlagt. An einem Abwärtseingang 50 liegt die Differenzfrequenz $\Delta f$ von der Frequenzauswerterschaltung 42. Der Zählerstand des Zählers 46 ist auf einen Digital-Analog-Wandler 52 aufgeschaltet. Der spannungsgesteuert-frequenzveränderliche Oszillator 12 ist über den Steuereingang 14 durch das analoge Ausgangssignal des Digital-Analog-Wandlers 52 gesteuert.

Es entsteht so ein geschlossener Regelkreis, in welchem durch Veränderung der Frequenz des Oszillators 12 die Differenzfrequenz $\Delta f$ einer ggf. kontrolliert veränderbaren.Sollfrequenz nachgeführt wird. Stimmen Differenzfrequenz und Sollfrequenz überein, dann erscheint zu jedem Impuls von der Frequenzauswerterschaltung 42, der auf den Abwärtseingang 50 des Zählers 46 gegeben wird und den Zählerstand um eines vermindert, ein Impuls vom Ausgang 40 des Mikroprozessors, wobei dieser Impuls auf den Aufwärtseingang 48 des Zählers 46 gegeben wird und den Zählerstand wieder um eines erhöht.

Der Zählerstand bleibt daher praktisch konstant. Ebenso bleibt dann auch das analoge Ausgangssignal des Digital-Analog-Wandlers 52 konstant.

Die Kapazität des Zählers 46 ist begrenzt. Der Oszillator 12 sollte daher wenigstens ungefähr auf die Frequenz des festfrequenten Oszillator 10 eingestellt sein, so daß der Zähler 46 und der Digital-Analog-Wandler 52 nur die Frequenzabweichungen zu steuern brauchen.

Zu diesem Zweck ist während eines Abgleichvorganges ein Rücksetzeingang 54 des Zählers 46 durch ein Rücksetzsignal ständig ansteuerbar, so daß der Zählerstand auf null gehalten wird. Der Abgleichvorgang wird von dem Mikroprozessor 38 eingeleitet, wie noch beschrieben wird. Das Rücksetzsignal erscheint dann an einem Ausgang 56 des Mikroprozessors 38. Das analoge Ausgangssignal des Digital-Analog-Wandlers 52 ist dann ebenfalls null.

Dem analogen Ausgangssignal des Digital-Analog-Wandlers 52 ist in einem Summierpunkt 58 eine veränderbare Spannung von einem Spannungsgeber 60 überlagerbar. Der Spannungsgeber 60 enthält einen Digital-Analog-Wandler 62. Auf den Digitaleingang des Digital-Analog-Wandlers 62 sind ansteigende oder abfallende Digitalwerte aufschaltbar. Diese Digitalwerte werden von dem Mikroprozessor 38 geliefernt und erscheinen an einem Ausgang 64.

Die Frequenzauswerterschaltung 42 gibt an einem Ausgang 66 ein Ausgangsignal bei Vorzeichenumkehr der Differenzfrequenz ab, d.h. wenn die Frequenz $f_2$ des Oszillators 12 durch die Frequenz $f_1$ des Oszillators 10 hindurchgeht. Durch dieses Ausgangssignal der Frequenzauswerterschaltung 42, das auf einen Eingang 68 des Mikroprozessors 38 geschaltet ist, wird einmal die.Spannungsveränderung des Spannungsgebers 60 unterbrochen und zum anderen das Rücksetzsignal am Ausgang 56 abgeschaltet.

Bei Inbetriebnahme der Vorrichtung arbeitet der Spannungsgeber 60 mit ansteigend-veränderbarer Spannung. Das analoge Ausgangssignal des Digital-Analog-Wandlers 52 wird durch das Rücksetzsignal am Eingang 54 des Zählers 40 auf null gehalten. Die Digitalwerte am Ausgang 64 des Mikroprozessors 38 steigen an. Entsprechend steigt die analoge Ausgangsspannung des Digital-Analog-Wandlers 62. Diese Ausgangsspannung wird über den Summierpunkt 58 ebenfalls am Steuereingang 14 des spannungsgesteuert-frequenzveränderlichen Oszillators 12.wirksam. Die Frequenz $f_2$ des Oszillators 12 steigt an. Bei einem Vorzeichenwechsel der Differenzfrequenze $\Delta f$, d.h. wenn $f_1 = f_2$, wird der Anstieg der Ausgangsspannung des Digital-Analog-Wandlers 62 unterbrochen. Die beiden Oszillatoren 10 und 12 haben jetzt gleiche Frequenzen. Gleichzeitig wird aber das Rücksetzsignal vom Eingang 54 weggenommen. Der Zähler 46 beginnt zu zählen. Es zählt aufwärts mit der Sollfrequenz vom Eingang 40 des Mikroprozessors. Damit steigt der Zählerstand und die Ausgangsspannung des Digital-Analog-Wandlers 52. Diese Ausgangsspannung addiert sich in dem Summierpunkt 58 zu der jetzt festen Ausgangsspannung des Digital-Analog-Wandlers 62. Die Frequenz $f_2$ des Oszillators 12 steigt an. Damit tritt eine Differenzfrequenz $\Delta$ f auf, die als Rückführung abwärts in den Zähler 46 eingezählt wird.Im stationären Zustand ist die Differenzfrequenz $\Delta$ f gleich der Sollfrequenz am Ausgang 40 des Mikroprozessors 38. Diese Differenzfrequenz $\Delta$ f erscheint dann auch als Schwebungsfrequenz im Bereich 36.

Die Kapazität des Zählers 46 kann im Betrieb dadurch überschritten werden, daß die Frequenzen der beiden Oszillatoren 10 und 12 durch irgendwelche Einflüsse auseinanderlaufen und durch die Regelschleife keine Gleichheit von Differenzfrequenz und Sollfrequenz herstellbar ist. In diesem Fall läuft der Zähler 46 entweder voll oder leer. Es tritt dann entweder ein Überlaufsignal an einem Überlaufausgang 70 oder ein Leerzählsignal an einem Leerzählausgang 72 des Zählers 46 auf, wenn vorgegebene Zählerstände über - bzw. unterschritten werden. Das Überlaufsignal ist auf einen Eingang 74 des Mikroprozessors 38 geschaltet. Das Leerzählsignal ist auf einen Eingang 76 des Mikroprozessors 38 geschaltet. Der Spannungsgeber 60 und das Rücksetzsignal am Ausgang 56 sind von den Signalen des Überlaufausgangs 70 und des Leerzählausgangs 72 so ansteuerbar, daß bei Auftreten eines Signals an dem Überlaufausgang 70 ein Abgleichvorgang der oben beschriebenen Art mit ansteigend veränderbarer Spannung am Digital-Analog-Wandler 62 und bei Auftreten eines Signals an dem Leerzählausgang 72 einen Abgleichvorgang mit veränderbarer Spannung eingeleitet wird.

Auf diese Weise erfolgt ständig eine automatische Nachstellung des Arbeitspunktes der Regelung.

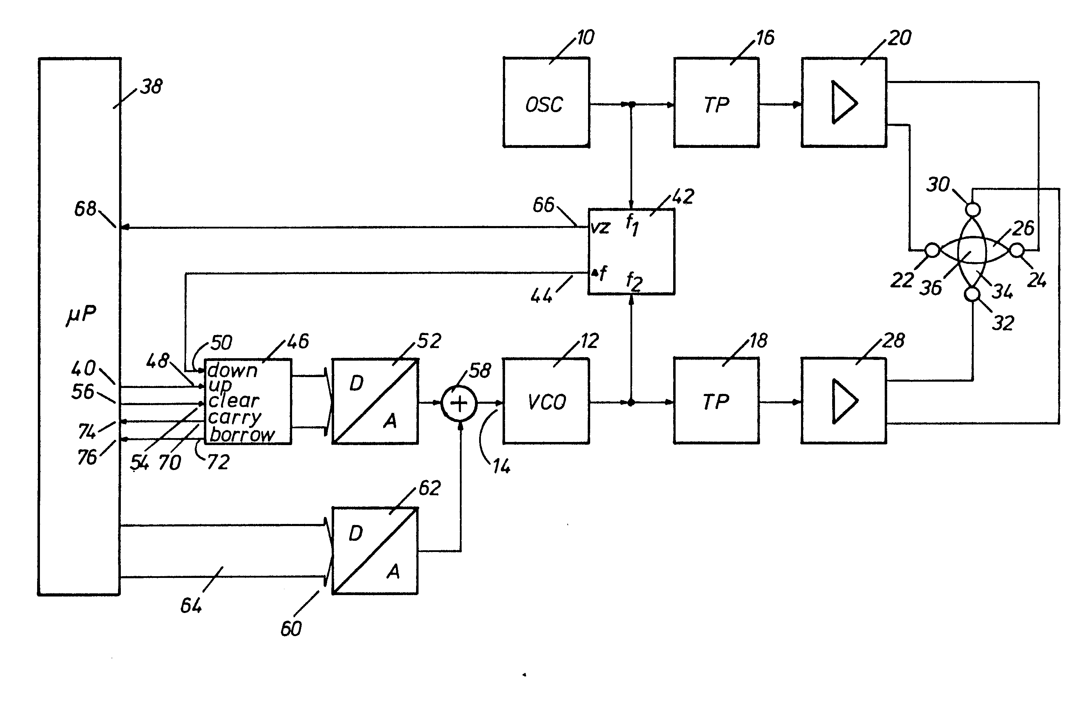

## Ansprüche

1. Vorrichtung zur Erzeugung von Strömen für die elektrotherapeutische Behandlung, bei welcher die verstärkten Ausgangsströme zweier gegeneinander verstimmter Mittelfrequenzoszillatoren auf Strompfaden, die einander im Behandlungsgebiet kreuzen, in ein Gewebe eingeleitet werden, so daß in dem Behandlungsgebiet Ströme mit einer Schwebungsfrequenz entstehen, enthaltend:
(a) einen festfrequenten Oszillator (10) und
(b) einen spannungsgesteuert-frequenzveränderlichen Oszillator (12) mit einem Steuereingang,
dadurch gekennzeichnet, daß
(c) die Ausgangsfrequenzen $(f_1, f_2)$ des festfrequenten Oszillators (10) und des spannungsgesteuert-frequenzveränderlichen Oszillators (12) auf eine Frequenzauswerterschaltung (42) aufgeschaltet sind, die eine Signalfolge mit Differenzfrequenz ($\Delta$ f) liefert,
(d) ein auf-und abwärtszählender Zähler (46) an einem Aufwärtseingang (48) mit einer Sollfrequenz und an einem Abwärtseingang (50) mit der Differenzfrequenz ($\Delta$ f) von der Frequenzauswerterschaltung (42) beaufschlagt ist,
(e) der Zählerstand des Zählers (46) auf einen Digital-Analog-Wandler (52) aufgeschaltet ist und
(f) der spannungsgesteuert-frequenzveränderliche Oszillator (12) durch das analoge Ausgangssignal des Digital-Analog-Wandlers (52) gesteuert ist.

2. Vorrichtung nach Anspruch 1, dadurch gekennzeichnet, daß
(a) während eines Abgleichvorganges ein Rücksetzeingang (54) des Zählers (46) durch ein Rücksetzsignal ständig ansteuerbar ist, so daß der Zählerstand auf null gehalten wird,
(b) dem analogen Ausgangsignal des Digital-Analog-Wandlers (52) eine veränderbare Spannung von einem Spannungsgeber (60) überlagerbar ist,
(c) die Frequenzauswerterschaltung (42) ein Ausgangssignal bei Vorzeichenumkehr der Differenzfrequenz abgibt,
(d) durch dieses Ausgangssignal der Frequenzauswerterschaltung (42)
-die Spannungsveränderung des Spannungsgebers (60) unterbrochen und
-das Rücksetzsignal abgeschaltet wird.

3. Vorrichtung nach Anspruch 2, dadurch gekennzeichnet, daß bei Inbetriebnahme der Vorrichtung der Spannungsgeber (60) mit ansteigend-veränderbarer Spannung arbeitet.

4. Vorrichtung nach Anspruch 2 oder 3, dadurch gekennzeichnet, daß
(a) der Zähler (46) einen Überlaufausgang (70) aufweist, an welchem ein Signal bei Überschreiten eines vorgegebenen Zählerstandes erscheint,
(b) der Zähler (46) weiterhin einen Leerzählausgang (72) aufweist, an welchem ein Signal bei Unterschreiten eines vorgegebenen Zählerstandes erscheint,
(c) der Spannungsgeber (60) und das Rücksetzsignal von den Signalen des Überlaufausganges (70) und des Leerzählausganges (72) so ansteuerbar ist, daß
-bei Auftreten eines Signals an dem Überlaufausgang (70) ein Abgleichvorgang mit ansteigend veränderbar Spannung und
-bei Auftreten eines Signals an dem Leerzählausgang (72) ein Abgleichvorgang mit absinkend veränderbarer Spannung eingeleitet wird

5. Vorrichtung nach einem der Ansprüche 2 bis 4, dadurch gekennzeichnet, daß der Spannungsgeber (60) einen Digital-Analog-Wandler (62) enthält, auf dessen Digitaleingang ansteigende oder abfallende Digitalwert aufschaltbar sind,

6. Vorrichtung nach einem der Ansprüche 1 bis 5, gekennzeichnet, durch einen Mikroprozessor (38)

-zur Steuerung des Ablaufs und

-zur Erzeugung der Sollfrequenz.

7. Vorrichtung nach den Ansprüchen 5 und 6, dadurch gekennzeichnet, daß die ansteigenden oder abfallenden Digitalwerte von dem Mikroprozessor (38) geliefert werden.